Europäisches Patentamt

European Patent Office

Office européen des brevets

(11) Publication number: **0 296 574**
**A2**

## (12) EUROPEAN PATENT APPLICATION

(21) Application number: 88109953.5

(51) Int. Cl.⁴: **C07K 7/06 , A61K 37/02**

(22) Date of filing: **22.06.88**

(30) Priority: **22.06.87 US 65241**

(43) Date of publication of application:
**28.12.88 Bulletin 88/52**

(84) Designated Contracting States:
**AT BE CH DE ES FR GB GR IT LI LU NL SE**

(71) Applicant: **F. HOFFMANN-LA ROCHE & CO.**
**Aktiengesellschaft**

**CH-4002 Basel(CH)**

(72) Inventor: **Danho, Waleed**
**118 Bunker Hill Road**
**Wayne, N.J. 07470(US)**
Inventor: **Madison, Vincent Stewart**
**12 Ronarm Drive**
**Mountain Lakes, N.J. 07046(US)**
Inventor: **Triscari, Joseph**
**22 Evergreen Drive**
**North Caldwell, N.J. 07006(US)**

(74) Representative: **Lederer, Franz, Dr. et al**
**Van der Werth, Lederer & Riederer**
**Patentanwälte Lucile-Grahn-Strasse 22**
**D-8000 München 80(DE)**

(54) **Cholecystokinin analogs for controlling appetite.**

(57) The invention relates to peptides of the general formula

$$X\text{-}(R)_n\text{-}Tyr(SO_3H)\text{-}(R^1)_p\text{-}R^2\text{-}R^3\text{-}R^4\text{-}R^5\text{-}N\text{-}methyl\text{-}Phe\text{-}Y \qquad I$$

wherein

$X$ = $-CO\text{-}R^6$, $\underline{-CO\text{-}OR^6}$, $\underline{-CO\text{-}(CH_2)_m\text{-}CH_3}$, $-CO\text{-}CO\text{-}OR^7$, or $-CO\text{-}(CH_2)_q\text{-}CO\text{-}OR^8$

$Y$ = $-OR^8$ or $\overline{-NR^9R^{10}}$

$R$ = Asp, Aeg

$R^1$ = Met, Nle, Leu or Ile

$R^2$ = Gly, Ala, D-Ala, or $\beta$-Ala

$R^3$ = Trp or Trp(For)

$R^4$ = Met, $\underline{Nle}$ or $\underline{Nva}$

$R^5$ = $Thr(SO_3H)$, $Ser(SO_3H)$, or $Hyp(SO_3H)$

$R^6$ = H or $C_{1-3}$-alkyl,

$R^7$ = H, $C_{1-3}$-alkyl, or halo-$C_{1-3}$-alkyl

$R^8$ = H or $C_{1-3}$-alkyl

$R^9$ = H or $C_{1-3}$-alkyl

$R^{10}$ = H or $C_{1-3}$-alkyl

$n$ = 0 or 1

$p$ = $\underline{0}$ or 1

$m$ = $\overline{3}$-14

$q$ = 1-3 or $\underline{4}$-14

with the proviso that at least one of the variables X, $R^4$, p and q represent a residue which is emphasized

and pharmaceutically acceptable salts thereof with food intake suppressing or appetite regulating activity.

Such compounds can be prepared by treating the appropriate unsulfated peptides with a sulfating agent or treating the corresponding formylated peptides with a deformylating agent, and if desired, converting such a peptide obtained into pharmaceutically acceptable salts.

## Cholecystokinin Analogs for Controlling Appetite

In all countries which have reached some prosperity obesity is one of the factors of increasing costs in health care. There are for example approximately 34 million Americans at least 20 percent above their desirable weights for whom treatment is advisable, according to the conclusions reached by a recent NIH concensus conference. (National Institutes of Health consensus Panel Report, February 13, 1985; See also Science, 1985, 207, 1019-1020.)

In these individuals obesity is a contributory factor to the increased incidence of cardiovascular disease, hypertension, hypercholesterolemia, non-insulin dependent diabetes (NIDD) and cancers of the uterus, breast, gall-bladder, colon, rectum and prostate. In addition obesity has a negative weight related impact on mortality; such that in extreme or morbid obesity the mortality ratio may be 1200 percent above normal.

Weight reduction is often recommended as the first course of action for patients suffering from NIDD, hypertension, hypercholesterolemia, coronary artery heart disease, gout and osteoarthritis. However, there are relatively few therapeutic tools which the physician can use to accomplish weight loss. Pharmaceutical agents which are currently used as adjuncts to dietary counseling are effective for short term therapy, but are unacceptable for long term use because of the development of tolerance, their CNS activity and undesirable side effects. Thus, approximately 95% of those patients who successfully lose weight return to their initial body weights within 12 to 84 months. An agent which reduces food intake by mimicking the body's own peripheral satiety signals would be expected to be more successful for use in chronic therapy, have fewer adverse side effects and be free of CNS activity.

Cholecystokinin (CCK) is a polypeptide hormone which was first isolated as a 33-amino acid peptide from the porcine gastrointestinal tract [Mutt et al., Biochem. J. (Proced. Biochem. Soc.) 1971, 125,57p; Mutt et al., Clin. Endocrinol., supplement, 1976, 5,175]. Peripherally administered CCK has been shown to produce satiety in the rat and the monkey and infusions of CCK-8, the octapeptide analog of CCK, has been shown to decrease food intake in lean and obese men (Smith J., Int. J. Obesity 1984, 8, Supplement 1,35; Jorpes et al., Acta Chem. Scand., 1964, 18,2408; Della-Fera et al., Science, 1979, 206,471; Gibbs et al., 1973, J. Comp. Physiology and Psychology, 84, 488). It is now accepted that CCK has satiety-inducing effects and thus, may be useful to reduce or suppress food intake in man.

The polypeptide hormone, CCK-33, has the amino acid sequence:

$$
\begin{array}{ccc}
1 & 5 & 10 \\
\end{array}
$$
Lys-Ala-Pro-Ser-Gly-Arg-Val-Ser-Met-Ile-
$$
\begin{array}{cc}
15 & 20 \\
\end{array}
$$
Lys-Asn-Leu-Gln-Ser-Leu-Asp-Pro-Ser-His-
$$
\begin{array}{cc}
25 & 30 \\
\end{array}
$$
Arg-Ile-Ser-Asp-Arg-Asp-Tyr($SO_3$H)-Met-Gly-Trp-
$$
33
$$
Met-Asp-Phe-$NH_2$.

Fragments of CCK, e.g. CCK-8 and CCK-7 also have been shown to have satiety-inducing effects. CCK-8 has the amino acid sequence:

$$
\begin{array}{cccccccc}
26 & 27 & 28 & 29 & 30 & 31 & 32 & 33 \\
\end{array}
$$
Asp-Tyr($SO_3$H)-Met-Gly-Trp-Met-Asp-Phe-$NH_2$.

CCK-7 is one amino acid less than CCK-8, i.e., it is CCK-8 minus the 26-position Asp.

Various CCK-8 analogs are known. For example, U.S. Pat. No. 4,400,377 teaches the use of analogs of CCK-8 to treat pain. U.S. Pat. No. 4,490,364 discloses that analogs of CCK-8 stimulate the contraction of the gall bladder and assist the secretion of gastric acid. The psychodepressant use of CCK-8 analogs is disclosed by U.S. Pat. No. 4,517,180.

Although various analogs of CCK-8 are known, neither the CCK derivatives of the invention, nor their use to suppress food intake in animals are known. Further, although the naturally occurring peptides, CCK-33, CCK-8 and CCK-7 have satiety inducing effects, these peptides are short acting. Accordingly, it is an object of the invention to prepare CCK-analogs which exhibit satiety-inducing effects which are equal to or greater than the naturally occurring CCK-8 but which also have longer lasting effects.

The following abbreviations or symbols are used to represent amino acids, active groups, protecting groups and the like.

| Symbol | Meaning |
|--------|---------|
| AA | Amino acid |
| Ac | Acetyl |
| Suc | Succinyl |
| Mal | Malonyl |
| For | Formyl |
| Aeg | Aminoethylglycine |
| Boc | tert. Butyloxycarbonyl |

Amino acids are given their commonly understood three-letter designation herein, unless otherwise specified the L-isomer is meant.

The invention is directed to peptides of the formula:

$X-(R)_n-Tyr(SO_3H)-(R^1)_p-R^2-R^3-R^4-R^5-N-methyl-Phe-Y$     I

wherein

$X = -CO-R^6$, $\underline{-CO-OR^6}$, $-CO-(CH_2)_m-CH_3$, $-CO-CO-OR^7$, or $-CO-(CH_2)_q-CO-OR^8$

$Y = -OR^8$ or $-NR^9R^{10}$

$R = Asp, Aeg$

$R^1 = Met, Nle, Leu$ or $Ile$

$R^2 = Gly, Ala, D-Ala,$ or $\beta-Ala$

$R^3 = Trp$ or $Trp(For)$

$R^4 = Met, \underline{Nle}$ or $\underline{Nva}$

$R^5 = Thr(SO_3H), Ser(SO_3H),$ or $Hyp(SO_3H)$

$R^6 = H$ or $C_{1-3}-alkyl,$

$R^7 = H, C_{1-3}-alkyl,$ or $halo-C_{1-3}-alkyl$

$R^8 = H$ or $C_{1-3}-alkyl$

$R^9 = H$ or $C_{1-3}-alkyl$

$R^{10} = H$ or $C_{1-3}-alkyl$

$n = 0$ or $1$

$p = \underline{0}$ or $1$

$m = 3-14$

$q = 1-3$ or $\underline{4-14}$

with the proviso that at least one of the variables $X$, $R^4$, $p$ and $q$ represent a residue which is emphasized and pharmaceutically acceptable salts thereof.

As used herein, the term "$C_{1-3}alkyl$" refers to methyl, ethyl, n-propyl and isopropyl. The term "halo" refers to chloro, bromo, fluoro and iodo. The term "$halo-C_{1-3}-alkyl$" refers to such groups as chloromethyl, bromomethyl, trifluoromethyl, chloroethyl, dichloroethyl, chloropropyl, bromoethyl, iodomethyl and the like.

Particularly preferred peptides of the present invention are:

$Ac-Tyr(SO_3H)-Nle-Gly-Trp-Nle-Thr(SO_3H)-N-methyl-Phe-NH_2$

and

$Ac-Tyr(SO_3H)-Gly-Trp-Met-Thr(SO_3H)-N-methyl-Phe-NH_2$.

The invention is further directed to compositions for suppressing food intake in animals comprising a food intake suppressing amount of the peptides of formula I, or of their pharmaceutically acceptable salts. Such compositions may be prepared in a conventional way and may contain conventional carriers and/or diluents.

Still another aspect of the invention is directed to a method of suppressing food intake in mammals which comprises administering to said mammal a food intake suppressing amount of the peptides of formula I or of their pharmaceutically acceptable salts. By mammal is meant any warm-blooded animal including human beings.

As used herein the term "food intake suppressing amount" refers to the amount of peptide (on a weight basis) per Kg of body weight of the animal which must be administered to suppress food intake. It is within the skill of the art to calculate such amounts considering the method of administration, the particular animal and the weight of the animal. The level of skill in the art relating to the use of CCK-8 as a satiety agent is illustrated by the references summarized by Morley, J. E. [Minireview-The Ascent of Cholecystokinin (CCK) From Gut to Brain, Life Sciences, 1982, 30, 479].

The polypeptides of formula I may be administered as water soluble salts, generally as salts of alkaline metals such as sodium or potassium salts, as alkylamine salts, preferably diethylamine salts or as acid addition salts, preferably the hydrochloride.

The peptides of formula I may be administered either to mammals in a variety of dosage forms e.g., intravenously, intraperitoneally, sublingually, rectally, intranasally, buccally or transdermally.

When formulated for injection, peptides of the invention and salts thereof are preferably presented as a sterile powder to be reconstituted with water or other suitable carriers shortly before administration. For example, for intravenous injections, sterile aqueous isotonic solutions may be used, preferably sodium chloride isotonic aqueous solution. The sterile powder is conveniently obtained by means of lyophilization; in that case the active ingredient is conveniently admixed with an inert carrier, such as lactose.

The peptides of the invention may be prepared using solid phase synthesis by the method generally described by Merrifield, J. Am. Chem. Soc., 85,2149 (1963) although other equivalent chemical synthesis known in the art may also be used. Solid-phase synthesis is commenced from the C-terminal end of the peptide which is to be synthesized by coupling a protected $\alpha$-amino acid by an amide bond to a suitable resin, e.g., benzylhydrylamine (BHA) or methylbenzylhydrylamine resin (MBHA). BHA and MBHA resin supports are commercially available and generally used when the peptide to be synthesized has an unsubstituted amide at the carboxy terminus.

All solvents used in the preparations described herein, e.g. methylene chloride ($CH_2Cl_2$), 2-propanol, and dimethylformamide (DMF) were Burdick & Jackson "Distilled in Glass" grade and used without additional distillation. Trifluoroacetic acid (TFA), diisopropylethylamine (DIPEA), and dicyclohexylcarbodiimide (DCC) were purchased from Chemical Dynamics Corp. and were "sequential" grade purity. 1,2-ethanedithiol (EDT) was purchased from Sigma Chemical Co. and used without further purification. All protected amino acids were of the L configuration unless otherwise indicated and were obtained from Bachem.

In solid phase synthesis methods, the reactive side chain groups of the various amino acid moieties are typically protected with suitable protecting groups which will prevent a chemical reaction from occuring at that site until the protecting group is ultimately removed. While specific protecting groups are disclosed in regard to the solid phase synthesis aspect, it should be noted that each amino acid can be protected by any protective groups conventionally used for the respective amino acid in solution phase synthesis. Among such protecting groups there are included, for example, conventional protecting groups for carboxyl groups selected from esters such as aryl esters, particularly phenyl or phenyl substituted with lower alkyl, halo, nitro, thio or lower($C_{1-7}$)alkylthio. All $\alpha$-amino groups were blocked with Boc functions. Side chain groups were substituted as follows: Asp, Ser, Thr with benzyl; Trp with formyl and Tyr with 2,6-dichlorobenzyl; Aeg with the benzyloxycarbonyl group. Purity of these compounds was confirmed by thin layer chromatography (TLC) and optical rotation. The benzhydrylamine (BHA) resin was a copolymer of styrene and 1% divinylbenzene in bead form (200-400 mesh) obtained from Beckman Instruments. Total nitrogen content was 0.654 meq/g.

The following instrumentation was utilized. Thin layer chromatography (TLC) was performed on glass backed precoated silica gel 60 F254 plates (Merck) using appropriate solvent systems. Detection of spots was by UV fluorescence quenching (254 nm absorption), iodine staining, or ninhydrin staining (for primary and secondary amines).

For amino acid analyses, peptides were hydrolyzed in 6N HCl containing phenol at 115°C for 24 hours in evacuated Reacti-Therm hydrolysis tubes (Wheaton Scientific Company, Milville, N.J. 08332). Analyses were performed on a Beckman 121M amino acid analyzer.

High performance liquid chromatography (HPLC) was conducted on a laboratory data control (LDC) apparatus consisting of a Constametric I pump, a Constametric III pump, a Gradient Master solvent programmer and mixer, and a Spectromonitor III variable wavelength UV detector. Analytical HPLC was performed with Waters Micro Bondapack $C_{18}$ reversed phase columns (0.4 x 25 cm). Preparative HPLC separations were run on a Partisil M20 10/50 ODS-3 (2.5x50 cm) column, or micro Bondapack $C_{18}$ (2.3x30 cm) column; in both cases, a pre-column of Whatman Co:Pell ODS pellicular packing was used.

The peptides were assembled in a stepwise manner on a solid support using a Vega 250 peptide synthesizer. The chemistry module was controlled by a Model 300 Microprocessor from Vega Biochemicals with manual operations at step 16 and 20.

Boc-N-methyl-Phe was coupled to the benzylhydrylamine resin according to methods known in the art preferably by a method as outlined in Example 1. Loading was determined by amino acid analysis. Any unreacted amino groups were capped by conventional reagents preferably with acetic anhydride and diisopropylethylamine.

The synthesis was started with resin and portions of peptide resin were removed at various points for separate analog preparations. The protocol for a typical synthetic cycle was as follows:

| Step | Reagent | Time |
|---|---|---|
| 1 | 1% EDT/$CH_2Cl_2$ | 1x30 sec |
| 2 | 50% TFA/$CH_2Cl_2$ 1% EDT | 1x1 min |
| 3 | Repeat Step 1 | |
| 4 | 50% TFA/$CH_2Cl_2$ 1% EDT | 1x15 min |
| 5 | $CH_2Cl_2$ | 1x30 sec |
| 6 | 2-Propanol | 1x30 sec |
| 7-8 | Repeat steps 5 & 6 | |
| 9 | $CH_2Cl_2$ | 2x30 sec |
| 10 | 8% DIPEA | 2x2 min |
| 11-15 | Repeat step 5-9 | |
| 16 | 5 equiv. Boc-AA anhydride | 1x30 min |
| 17 | 1% DIPEA | 1x5 min |
| 18-19 | Repeat steps 6&9 | |
| 20-21 | Repeat steps 16&17 if Kaiser test is positive | |
| 22 | 2-Propanol | 1x30 sec |
| 23-24 | Repeat steps 5&6 | |
| 25 | $CH_2Cl_2$ | 1x30 sec |
| 26 | DMF | 2x30 sec |
| 27 | $CH_2Cl_2$ | 3x30 sec |

Solvents for all washings and couplings were measured to volumes of 10-20 ml/g resin. Couplings were perfcrmed as the symmetrical anhydrides of the Boc-amino acids. They were performed in $CH_2Cl_2$ at 0°C in 15 min. using 10 equivalents of Boc-amino acid and 5 equivalents of DCC.

Coupling reactions were monitored by the Kaiser ninhydrin test to determine whether coupling was complete after step 19 [Kaiser, E. et al., Anal. Biochem., 34, 595-598 (1970)]. Total cycle times ranged from 54-160 minutes per residue. In case of formylated peptides in order to maintain the formyl group said treatment can be performed in the following manner. To 5.3 g of the peptide-resin 12 ml of HF containing dimethylsulfide (31 ml) and p-cresol (4.6 ml) is added at 0°C for 1 hour. After evaporation to a low volume, fresh anhydrous HF (42 ml) is destilled into the reaction vessel for a second treatment for 2 hours at 0°C. Treatment is proceeded, in the same way as in the synthesis of non formylated peptides given in detail in example 1.

The fully assembled peptide-resins were dried under high vacuum overnight. Deblocking and cleavage conditions were the modified procedures of Tam et al. [Tetrahedron Letters (1982), 23, 4435] which were optimized for CCK-8 analogs generally. The peptide-resin was treated in a teflon HF apparatus (Peninsula) in a way given in detail in the examples. In case of formylated peptides in order to maintain the formyl group said treatment can be performed in the following manner. To 5.3 g of the peptide-resin 12 ml of HF containing dimethylsulfide (31 ml) and p-cresol (4.6 ml) is added at 0°C for 1 hour. After evaporation to a low volume, fresh anhydrous HF (42 ml) is destilled into the reaction vessel for a second treatment for 2 hours at 0°C. Treatment is proceeded in the same way as in the synthesis of nonformylated peptides given in detail in example 1.

Preparative purification were carried out directly with the crude unsulfated peptide by HPLC on a (2.3 x 30 cm) micro Bondapack $C_{18}$ or (2.5-50 cm) Whatman ODS-3 column. The peptides were applied in a minimum volume of 50% AcOH, and eluted with a slow gradient (4 hr) of 5-65%, 0.022% TFA/$CH_3CN$, at a flow rate of 8.0 ml/min. Fractions were collected at 3 minute intervals and cuts were made after inspection by analytical HPLC. Fractions, judged to be greater then 97% pure, were pooled and lyophilized.

Purity of the individual unsulfated peptides was checked by HPLC and was 99% in all cases. Amino acid analyses of the individual peptides were performed and the expected values were obtained in each case. U.V., I.R., and M.S. were also performed on the analogs confirming the chemical integrity of the peptides.

The sulfuric acid-ester-group containing peptides were prepared by double sulfation of the hydroxyl groups (tyrosine, serine, threonine, or hydroxyproline) using pyridine acetyl sulfuric acid reagent. A typical sulfation was carried out as follows: 60-240 mg of pyridine acetyl sulfate (PAS) was dissolved in 10 ml of pyridine and mixed at 60°C for 10 minutes. N-acetyl-CCK-8 analog (10 mg) is dissolved in 10 ml of pyridine and mixed with the PAS reagent. After heating and mixing for 45-60 min. at 60°C, it is neutralized with 2 volumes 0.05 M ammonium bicarbonate lyophilized and purified by HPLC.

The sulfated peptides were purified by preparative reverse phase HPLC on a $C_{18}$-10$\mu$ (ES Industries) (1.25 x 30 cm) column using a 2 hours gradient (10-40%) of acetonitrile in 0.05 M ammonium bicarbonate with a flow rate of 5 ml/mn and detection at 240 nm. Fractions to be pooled and peptide purity were determined by analytical HPLC using a Bondapack $C_{18}$-10 $\mu$ Waters column (0.30 x 30 cm), and an acetonitrile in ammonium bicarbonate gradient with a flow of 2 ml and detection at 215 nm.

The purity of the sulfated peptides were determined by analytical HPLC, amino acid analysis, UV, IR, MS and NMR.

The formyl group can be cleaved from the $N^1$-formyltryptophan containing CCK derivatives of the invention by adding 1 ml of 0.1N $NH_4OH$ (pH 10.5) to 1 mg of peptide for 1 to 4 hours at room temperature followed by lyophilization.

The following examples illustrate in detail the preparation of peptides with appetite regulating activity of the invention utilizing the procedures described above. In the examples described below, unless otherwise stated, peptides were characterized and the purity was determined using amino acid analysis, analytical HPLC, U.V., I.R. and M.S.

## Example 1

## Preparation of Ac-Tyr(SO$_3$H)-Nle-Gly-Trp-Nle-Thr(SO$_3$H)-N--methyl-Phe-NH$_2$

Boc-N-methyl-Phe (5g, 17.8 mmol) was dissolved in a mixture of 50 ml methylene chloride and 50 ml of dimethylformamide chilled to 0°C and with stirring (1.8g, 9 mmol) dicyclohexylcarbodiimide was added and the mixture was stirred for 60 minutes at 0°C.

Separately 5g of benzylhydrylamine resin (0.56 mmole N/g) (crosslinked copolymer of styrene with 1% divinylbenzene) was washed with 10% diisopropylethylamine in methylene chloride for 30 min, filtered and washed with methylene chloride, dimethylformamide, and methylene chloride. The chilled mixture above was added to the resin and stirred for 24 hours at room temperature. The resin was filtered, washed with methylene chloride, dimethylformamide, isopropanol, methylene chloride, dimethylformamide, isopropanol, and methylene chloride, and dried under high vacuum.

Amino acid analysis showed the resin to contain 0.20 mmoles of N-methylphenylalanine per gram of resin. Unreacted amino groups were capped by shaking the resin with 5 ml of acetic anhydride and 5 ml diisopropylethylamine in methylene chloride for 60 minutes. The resin was filtered and washed with methylene chloride, isopropanol, dimethylformamide and methylene chloride. Two grams (0.4 mmol) of Boc-N-methylphenylalanine resin was subjected to sequential solid phase synthesis using the procedure described above. All couplings were performed using the symmetrical anhydrides of Boc amino acids as described. At step 16 and 20 the activated amino acids were added with the corresponding reaction times as follows: six individual cycles were performed with Boc-O-benzyl-threonine (610 mg, 2 mmole, 60 min.; 610 mg, 2 mmole, 60 min.), Boc-norleucine (460 mg, 2 mmole, 30 min.; 460 mg, 2mmole, 30 min.), Boc-N$^1$-formyl-tryptophan (665 mg. 2 mmole, 30 min; 665 mg, 2 mmole, 30 min), Boc-glycine (350 mg, 2 mmole, 30 min.; 350 mg, 2 mmole, 30 min.), Boc-norleucine (460 mg, 2 mmole, 30 min.; 460 mg, 2 mmole, 30 min.) and Boc-2-6-dichlorobenzyl-tyrosine (880 mg, 2 mmole, 30 min; 880 mg, 2 mmole, 30 min).

Cleavage of the Boc-protecting groups and acetylation of the resin with 10 ml acetic anhydride, 10 ml of pyridine in methylene chloride for 60 min. yielded 2.93g of the acetylated-heptapeptidyl resin.

2.93 g of the peptidyl resin was cleaved by treatment with 6 ml of HF containing dimethylsulfide (20 ml), anisol (3 ml) and (1 ml) ethanedithiol for 1 hour at 0°C. After evaporation to a low volume fresh anhydrous HF (15 ml) was distilled into the reaction vessel for a second treatment for 1 hour at 0°C. After thorough evaporation the resin was washed with ethylacetate, then triturated with 4 x 15 ml of 50% acetic acid filtered and lyophilized to yield 480 mg of crude peptide. 85 mg of the crude peptide was purified by preparative HPLC on a (2.3 x 30 cm) micro Bondapack $C_{18}$ column. The peptide was eluted with a linear gradient of 5 to 65%, 0.22% TFA/$CH_3CN$ at a flow rate of 8 ml/min., detection was at 280 nm. The main peak was collected and lyophilized to yield 22mg (31%) Ac-Tyr-Nle-Gly-Trp-Nle-Thr-N-methyl-Phe-$NH_2$. This material was homogenous by HPLC and gave the correct amino acid analysis.

To 60 mg of pyridine acetyl sulfate (PAS) was added 10 ml of dry distilled pyridine. The resulting mixture was heated at 60°C with stirring for 10 min. The solution was allowed to cool, and 10 mg of Ac-Tyr-Nle-Gly-Trp-Nle-Thr-N-methyl-Phe-$NH_2$ dissolved in 10 ml of pyridine was added to the solution and the reaction mixture was stirred for 1 hour at 60°C. Thereafter, the reaction mixture was neutralized with 2 volumes of ammonium bicarbonate and lyophilized. Purification was achieved by preparative reverse phase HPLC on an ES Industries $C_{18}$-10 micron column (1.25 x 30 cm) using a linear gradient of 10-40% of 0.05 M $NH_4HCO_3$/ $CH_3CN$ in 120 min. with a flow rate of 5 ml/min and detection at 240 nm. The yield was 8 mg (66%) of Ac-Tyr-($SO_3H$)-Nle-Gly-Trp-Nle-Thr($SO_3H$)-N-methyl-Phe-$NH_2$. This material was homageneous by HPLC and gave the following amino acid analysis: Asp 0.90 (1); Gly 1.00 (1); Nle 2.00; Tyr 1.00 (1); Trp 0.60 (1): N-methyl-Phe. n.d.

## Example 2

### Preparation of Ac-Tyr($SO_3H$)-Gly-Trp-Met-Thr($SO_3H$)-N-methyl-Phe-$NH_2$

1.5 g (0.3 mmol) of Boc-N-methylphenylalanine resin which was obtained in the same way as in Example 1 was subjected to sequential solid phase synthesis using the same procedure as in Example 1. All couplings were performed using the symmetrical anhydrides of Boc-amino acids as described before. At step 16 and 20 the activated amino acids were added with the corresponding reaction times as follows: five individual cycles were performed with Boc-O-benzyl-threonine(610 mg, 2 mmole, 60 min; 610 mg, 2 mmol, 60 min), Boc-methionine (500 mg, 2 mmole, 30 min; 500 mg, 2 mmole, 30 min), Boc-$N^1$-formyl tryptophan (665 mg, 2 mmole, 30 min.; 665 mg, 2 mmole, 30 min). Boc-glycine (350 mg, 2 mmole, 30 min.; 350 mg, 2 mmole, 30 min.), and Boc-2,6- dichlorobenzyl-tyrosine (880 mg, 2 mmole, 30 min.; 880 mg, 2 mmole, 30 min). Deprotection of the Boc-protecting group and acetylation of the resin with 8 ml acetic anhydride, 8 ml pyridine in methylene chloride for 60 min yielded 1.65g of the acetylated hexapeptide resin.

The resin was cleaved by treatment with HF containing dimethylsulfide, anisol, and ethanedithiol using the same procedure as in example 1. The resin was washed with ether and ethylacetate then triturated with 30% acetic acid, filtered and lyophilized to yield 110 mg of crude peptide.

110 mg of this crude peptide was purified by preparative HPLC on a (2.3 x 30 cm) micro Bondapack $C_{18}$ column. The peptide was eluted with a linear gradient of 5 - 65%, 0.022% TFA/$CH_3CN$ at a flow rate of 8 ml/min., detection was at 280 nm. The main peak was collected and lyophilized to yield 15 mg (11.6%) of Ac-Tyr-Gly-Trp-Met-Thr-N-methyl-Phe-$NH_2$. This material was homogenous by HPLC and gave the correct amino acid analysis.

15 mg of Ac-Tyr-Gly-Trp-Met-Thr-N-methyl-Phe-$NH_2$ were dissolved in 15 ml of pyridine and added to a solution of 240 mg of pyridine acetyl sulfate (PAS) in 20 ml of pyridine prepared in the same manner as described in Example 1. The reaction mixture was stirred for 1 h at 60°C then neutralized with 2 volumes of ammonium bicarbonate, and lyophilized. Purification was achieved by preparative HPLC using the same condition as described in Example 1. The yield was 11 mg (61%) of Ac-Tyr($SO_3H$)-Gly-Trp-Met-Thr($SO_3H$)-N-methyl-Phe-$NH_2$.

Amino acid analysis: Thr 0.86(1); Glu 1.00(1); Met 1.02(1); Tyr 1.01(1); Trp 0.72(1); N-methyl-Phe n.d.

## Example 3

## In Vitro Receptor Binding Assay

Frozen bovine striatum (approx. 5 g) and fresh rat pancreas (approx. 5 g) cleaned of fat and extraneous tissue were homogenized in HEPES buffer #1 (10 mM HEPES, 130 mM NaCl, 5 mM $MgCl_2$, pH 7.4) using 35 parts buffer per 1 part tissue on a wet weight/volume basis (approx. 175 ml). The tissue was homogenized 2 x for approx. 15 sec. at 0°C using a Polytron homogenizer at a setting of 6. The tissue was isolated by centrifugation at 48,000 x g for 10 min. at 0°C. The resulting tissue pellet was resuspended in HEPES buffer #2 [10 mM HEPES, 130 mM NaCl, 5 mM $MgCl_2$, 1 mg/L phenylmethanesulfonyl fluoride (PMSF), 200 ml/L Bacitracin]: 1 part striatal tissue (original wet weight) per 80 parts buffer and 1 part pancreas tissue (original wet weight) per 70 parts buffer.

Incubation was initiated by combining various concentrations of native CCK-8 or peptides of formula I with $^3$H-CCK-8-($SO_3$H) (final conc. = 0.15 nM) and tissue homogenate (striatum 0.26 mg protein in 2 ml final volume; pancreas 0.165 mg protein in 1 ml final volume). Samples were incubated for 30 min. at 25° C and the incubation terminated by pouring the mixture onto a pre-wet Whatman GF/B filter on a Sandbeck Vacuum Filtration Manifold. The incubation tubes were washed with 2 x 3 ml of ice-cold HEPES Buffer #2 and the wash filtered through the GF/B filter. The filter was air dried for 10 min. and then placed in a scintillation vial with 12 ml of Beckman HP/b Ready-Solv scintillation cocktail. The vials were shaken for 2 hours and then counted using a Beckman Model 7800 liquid scintillation spectrometer. Non-specific binding was determined in the pressure of 1 micromole native CCK-8 and subtracted from all samples to determine specific binding. The concentration of peptide necessary to inhibit 50% of total specific $^3$H-CCK-8-($SO_3$H) binding ($IC_{50}$ value) was determined by log-probit analysis.

The results are summarized in Table I.

## Example 4

## Two-Meal Feeding Assay

Male Sprague-Dawley (CD) rats weighing 180-200 grams (Charles River Breeding Laboratories) were acclimated to a 12 h light/dark cycle (6 a.m. to 6 p.m.) in a room kept at 22°C. They were subsequently fasted for two days, weighed, placed in individual cages, and a four-day period of meal training was begun. During this time the rats were given ground laboratory chow (Purina Lab Chow) in jars for one hour from 9:00 a.m. until 10:00 a.m., the jars were removed from 10:00 a.m. to 12:00 p.m., and placed back in the cages from 12:00 until 1:00 p.m. Under this '1-2-1' meal feeding regime, most rats learn to eat approximately as much per day during the two hours they have access to food as rats which have food ad libitum over the entire 24-hour day. On the fourth day, the rats were weighed again, and any which lost more than five grams body weight were excluded from the test. The animals were then distributed into experimental (n = 5 to 6) and control groups (n = 6-12), but not matched for body weight.

Peptides of the invention were suspended either in saline, if soluble, or in 1% gum arabic, if insoluble, at concentrations of 0 to 320 µg/ml/kg body weight and were administered intraperitonerally 15 min before the first meal on day 5 of meal feeding. The rats were then given their meals as they had been during the previous four days, and the food cups were weighed both before and after each meal to determine food consumption. Food intake was expressed as a mean and standard error of the mean in grams consumed or as a percent of control values for the various groups. The treated groups were compared to the control groups by t-test analysis. The results are summarized in Table I.

9

TABLE I

| Peptide | Example No. | Bovine Striatum (nM) | Rat Pancreas (nM) | Dose microgm/Kg | Food Intake | |
|---|---|---|---|---|---|---|
| | | | | | 1st Meal | 2nd Meal |
| | | | | | % of Control | |
| CCK-8 | | 1-3.2 | 1-4.6 | 32 | 27 ± 17*** | 149 ± 6*** |
| Ac-Tyr($SO_3H$)-Nle-Gly-Trp-Nle-Thr($SO_3H$)-N-methyl-Phe-$NH_2$ | 1 | 245 | 0.14 | 32 | 21 ± 12*** | 52 ± 13** |
| | | | | 16 | 27 ± 17** | 45 ± 11** |
| | | | | 10 | 13 ± 1*** | 62 ± 8 |
| | | | | 3 | 23 ± 13*** | 59 ± 6 |
| | | | | 1 | 36 ± 4*** | 71 ± 13 |
| Ac-Tyr($SO_3H$)-Gly-Trp-Met-Thr($SO_3H$)-N-methyl-Phe-$NH_2$ | 2 | 1600 | 70 | 320 | 4 ± 5*** | 121 ± 3 |
| | | | | 32 | 36 ± 8*** | 141 ± 9** |
| | | | | 3 | 86 ± 16 | 120 ± 13 |
| Values significantly different then their respective controls | | | | | | |

***p ≤0.001,
**p ≤0.01,
*p ≤0.05

EP 0 296 574 A2

EP 0 296 574 A2

**Claims**

1. A peptide of the formula

X-(R)$_n$-Tyr(SO$_3$H)-(R$^1$)$_p$-R$^2$-R$^3$-R$^4$-R$^5$-N-methyl-Phe-Y     I

wherein

X = -CO-R$^6$, -CO-OR$^6$, -CO-(CH$_2$)$_m$-CH$_3$, -CO-CO-OR$^7$, or -CO-(CH$_2$)$_q$-CO-OR$^8$

Y = -OR$^8$ or -NR$^9$R$^{10}$

R = Asp, Aeg

R$^1$ = Met, Nle, Leu or Ile

R$^2$ = Gly, Ala, D-Ala, or $\beta$-Ala

R$^3$ = Trp or Trp(For)

R$^4$ = Met, Nle or Nva

R$^5$ = Thr(SO$_3$H), Ser(SO$_3$H), or Hyp(SO$_3$H)

R$^6$ = H or C$_{1-3}$-alkyl,

R$^7$ = H, C$_{1-3}$-alkyl, or halo-C$_{1-3}$-alkyl

R$^8$ = H or C$_{1-3}$-alkyl

R$^9$ = H or C$_{1-3}$-alkyl

R$^{10}$ = H or C$_{1-3}$-alkyl

n = 0 or 1

p = 0 or 1

m = 3-14

q = 1-3 or 4-14

with the proviso that at least one of the variables X, R$^4$, p and q represent a residue which is emphasized and pharmaceutically acceptable salts thereof.

2. Ac-Tyr(SO$_3$H)-Nle-Gly-Trp-Nle-Thr(SO$_3$H)-N-methyl-Phe-NH$_2$

3. Ac-Tyr(SO$_3$H)-Gly-Trp-Met-Thr(SO$_3$H)-N-methyl-Phe-NH$_2$

4. A peptide according to any one of claims 1 to 3 for use as therapeutically active substance, particularly for controlling or suppressing food intake.

5. A process for the manufacture of a peptide according to any one of claims 1 to 3, which process comprises treating the corresponding unsulfated peptide with a sulfating agent, or treating the corresponding formylated peptide possibly linked to a carrier with a deformylating agent, and if desired, converting such peptide obtained into a pharmaceutically acceptable salt.

6. A pharmaceutical composition containing a peptide or a pharmaceutically acceptable salt thereof according to any one of claims 1-3 and a non-toxic, inert, therapeutically acceptable carrier material.

7. The use of a compound according to any one of claims 1-3 or of a composition according to claim 6 in the control or prevention of illness, especially for controlling or suppressing food intake.

Claims for the following contracting States: GR, ES

1. A process for the preparation of a peptide of the formula

X-(R)$_n$-Tyr(SO$_3$H)-(R$^1$)$_p$-R$^2$-R$^3$-R$^4$-R$^5$-N-methyl-Phe-Y     I

wherein

X = -CO-R$^6$, -CO-OR$^6$, -CO-(CH$_2$)$_m$-CH$_3$, -CO-CO-OR$^7$, or -CO-(CH$_2$)$_q$-CO-OR$^8$

Y = -OR$^8$ or -NR$^9$R$^{10}$

R = Asp, Aeg

R$^1$ = Met, Nle, Leu or Ile

R$^2$ = Gly, Ala, D-Ala, or $\beta$-Ala

R$^3$ = Trp or Trp(For)

R$^4$ = Met, Nle or Nva

R$^5$ = Thr(SO$_3$H), Ser(SO$_3$H), or Hyp(SO$_3$H)

R$^6$ = H or C$_{1-3}$-alkyl,

R$^7$ = H, C$_{1-3}$-alkyl, or halo-C$_{1-3}$-alkyl

R$^8$ = H or C$_{1-3}$-alkyl

R$^9$ = H or C$_{1-3}$-alkyl

R$^{10}$ = H or C$_{1-3}$-alkyl

11

n = 0 or 1

p = $\underline{0}$ or 1

m = $\underline{3\text{-}14}$

q = 1-3 or $\underline{4\text{-}14}$

with the proviso that at least one of the variables X, $R^4$, p and q represent a residue which is emphasized and of a pharmaceutically acceptable salts thereof which process comprises treating the corresponding unsulfated peptide with a sulfating agent, or treating the corresponding formylated peptide possibly linked to a carrier with a deformylating agent, and if desired, converting such peptide obtained into a pharmaceutically acceptable salt.

2. A process according to claim 1 for the preparation of a peptide of the formula
Ac-Tyr(SO$_3$H)-Nle-Gly-Trp-Nle-Thr(SO$_3$H)-N-methyl-Phe-NH$_2$

3. A process according to claim 1 for the preparation of a peptide of the formula
Ac-Tyr(SO$_3$H)-Gly-Trp-Met-Thr(SO$_3$H)-N-methyl-Phe-NH$_2$

4. A process for the preparation of a pharmaceutical composition, which process comprises mixing a peptide according to any one of claims 1 to 3 or a pharmaceutically acceptable salt thereof and, if desired, one or more other therapeutically active substances with a therapeutically inert carrier material and bringing the mixture into a galenical administration form.

5. A pharmaceutical composition containing a peptide according to any one of claims 1 to 3 or a pharmaceutically acceptable salt thereof and a therapeutically inert carrier material.

6. The use of a compound according to any one of claims 1 to 3 for the manufacture of a pharmaceutical composition according to claim 5.

7. A compound according to any one of claims 1-3 whenever prepared according to a process of any one of claims 1-3.

8. The invention as hereinbefore described.

9. A peptide of the formula

X-(R)$_n$-Tyr-(R$^1$)$_p$-R$^2$-R$^3$-R$^4$-R$^5$-N-methyl-Phe-Y     (I)

wherein

X = -CO-R$^6$, $\underline{\text{-CO-OR}^6}$, $\underline{\text{-CO-(CH}_2)_m\text{-CH}_3}$, -CO-CO-OR$^7$, or -CO-(CH$_2$)$_q$-CO-OR$^8$

Y = -OR$^8$ or -NR$^9$R$^{10}$

R = Asp, Aeg

R$^1$ = Met, Nle, Leu or Ile

R$^2$ = Gly, Ala, D-Ala, or $\beta$-Ala

R$^3$ = Trp or Trp(For)

R$^4$ = Met, $\underline{\text{Nle}}$ or $\underline{\text{Nva}}$

R$^5$ = Thr(SO$_3$H), Ser(SO$_3$H), or Hyp(SO$_3$H)

R$^6$ = H or C$_{1-3}$-alkyl,

R$^7$ = H, C$_{1-3}$-alkyl, or halo-C$_{1-3}$-alkyl

R$^8$ = H or C$_{1-3}$-alkyl

R$^9$ = H or C$_{1-3}$-alkyl

R$^{10}$ = H or C$_{1-3}$-alkyl

n = 0 or 1

p = $\underline{0}$ or 1

m = $\underline{3\text{-}14}$

q = 1-3 or $\underline{4\text{-}14}$

with the proviso that at least one of the variables X, $R^4$, p and q represent a residue which is emphasized and pharmaceutically acceptable salts thereof.